(19) Europäisches Patentamt
European Patent Office
Office européen des brevets

(11) **EP 4 227 073 A1**

(12) **EUROPEAN PATENT APPLICATION**
published in accordance with Art. 153(4) EPC

(43) Date of publication:
**16.08.2023 Bulletin 2023/33**

(21) Application number: **21877571.6**

(22) Date of filing: **04.10.2021**

(51) International Patent Classification (IPC):
**B29D 23/20** (2006.01)   **B29K 27/18** (2006.01)
**B29L 23/00** (2006.01)   **A61M 25/00** (2006.01)
**B29C 53/60** (2006.01)   **B29C 70/04** (2006.01)
**B29C 70/32** (2006.01)   **F16L 11/16** (2006.01)
**F16L 11/24** (2006.01)

(52) Cooperative Patent Classification (CPC):
**A61M 25/00; B29C 53/60; B29C 70/32;**
**B29D 23/20; F16L 11/16; F16L 11/24;** B29C 70/04;
B29K 2027/18; B29L 2023/00

(86) International application number:
**PCT/JP2021/036663**

(87) International publication number:
**WO 2022/075272 (14.04.2022 Gazette 2022/15)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **05.10.2020   JP 2020168783**
**07.10.2020   JP 2020169577**
**12.11.2020   JP 2020188829**

(71) Applicant: **Junkosha Inc.**
**Kasama-shi, Ibaraki 309-1603 (JP)**

(72) Inventors:
• **TANABE, Suguru**
**Kasama-shi Ibaraki 309-1603 (JP)**
• **YAMADA, Naoya**
**Kasama-shi Ibaraki 309-1603 (JP)**

(74) Representative: **Daub, Thomas**
**Patent- und Rechtsanwaltskanzlei Daub**
**Bahnhofstrasse 5**
**88662 Überlingen (DE)**

(54) **TUBE**

(57) The present invention relates to a tube comprising polytetrafluoroethylene, and an object of the present invention is to provide a tube having an appropriate tensile strength and flexibility and being excellent internal insertion property. The object can be achieved by a tube comprising polytetrafluoroethylene according to the present invention, in which, in a case where a surface shape of an inner surface of the tube is measured, when a direction of the measurement is parallel to a longitudinal direction of the tube and the measurement is performed in a forward direction and a reverse direction, a variation rate between a size of a periodic step measured in the forward direction and a size of the periodic step measured in the reverse direction is 5% or more.

*FIG. 1*

EP 4 227 073 A1

**Description**

**TECHNICAL FIELD**

The present invention relates to a tube using polytetrafluoroethylene (hereinafter, referred to as "PTFE").

**BACKGROUND ART**

There is a product provided with a fluororesin layer as an inner layer of a tubular body, such as a product for medical use and a fluid transport tube that requires chemical resistance. In particular, for uses requiring heat resistance, chemical resistance, cleanability, non-adhesiveness, low friction, and the like, PTFE is suitably used among fluororesins. For example, for the purpose of improving slidability of an inner surface of a catheter, a catheter in which a PTFE liner tube is disposed in an innermost layer of the catheter, etc. are known.

As the PTFE liner tube, for example, there is known a PTFE liner tube obtained by a method in which a core wire such as a metal wire is dip-coated with a PTFE dispersion, dried, and sintered, and then the core wire is pulled out, and molded (see, for example, Patent Literature 1). There is a method in which a paste obtained by mixing a PTFE powder and an organic solvent called an auxiliary agent is extruded onto a core wire, dried, and sintered, and then the core wire is pulled out, and molded (see, for example, Patent Literature 2). As a tube improved in mechanical strength, Patent Literature 3 discloses a tube obtained by a method in which a paste obtained by mixing a PTFE powder and an auxiliary agent is extruded into a tube shape and then a molded liner tube is stretched in a longitudinal direction to make the liner tube to be thin, and as a tube that is high in tensile strength and extensibility, Patent Literature 4 discloses a thin PTFE tube obtained by a devised molding method.

A catheter used for an intravascular surgery or the like needs to be percutaneously inserted into a body to pass through a blood vessel and cause a catheter distal end to reach a lesion site, and thus straightness for passing through the blood vessel straight, operation transmissibility for transmitting an operation of an operator, flexibility for reducing a burden on a patient, and the like are required. In order to satisfy all the requirements, the catheter is implemented by laminating layers having different characteristics, and among the layers, an innermost PTFE liner tube is required to be excellent in mechanical strength such as high tensile strength, extensibility, and crack resistance while being thin.

A treatment tool or the like used for treatment in the blood vessel is caused to be inserted into the inside of the catheter, and a thin inserted object (the treatment tool or the like) is required to be moved straight inside a narrow catheter, and thus it is desirable that the PTFE liner tube has a strength which allows the PTFE liner tube not to be damaged due to friction with the inserted object, and an inner surface of the PTFE liner tube is improved in operability of the inserted object. However, there was a problem that the PTFE liner tube molded by the method according to Patent Literature 1 or Patent Literature 2 was low in mechanical strength, and the tube according to Patent Literature 3 which was improved in tensile strength was high in tensile strength, but on the other hand, was poor in extensibility and flexibility. Patent Literature 4 discloses the thin PTFE tube that is high in tensile strength and extensibility, but even the tube could not be said to sufficiently satisfy requirements for uses requiring higher performance.

**PRIOR ART DOCUMENTS**

**PATENT LITERATURES**

Patent Literature 1: JP2000-316977A
Patent Literature 2: JP2013-176583A
Patent Literature 3: JP2004-340364A
Patent Literature 4: Japanese Patent No. 6244490

**SUMMARY OF INVENTION**

**OBJECT TO BE ACHIEVED BY THE INVENTION**

In view of the above-mentioned problem, an object of the present invention is to provide a thin tube using PTFE, which has an appropriate tensile strength and flexibility and is excellent in insertion property.

## MEANS FOR ACHIEVING THE OBJECT

The present object can be achieved by a PTFE tube, the tube comprising PTFE, in which, in a case where a surface shape of an inner surface of the tube is measured, when a direction of the measurement is parallel to a longitudinal direction of the tube and the measurement is performed in a forward direction and a reverse direction, a variation rate between a size of a periodic step measured in the forward direction and a size of the periodic step measured in the reverse direction is 5% or more.

The tube is preferably a tube in which at least one film is spirally wound, and which is formed by the at least one film without a gap. It is preferable that in the tube, one or more films are spirally wound clockwise, and one or more films are spirally wound counterclockwise.

The tube is preferably a PTFE tube having a 20% strain tensile strength obtained by a tensile test according to JIS K7127-1999 of 100 N/mm$^2$ or more.

The tube according to the present invention preferably has an average thickness of 5 $\mu$m or more and 75 $\mu$m or less, and has an inner diameter of 0.05 mm or more and 10 mm or less.

In the tube according to the present invention, a width of the periodic step is preferably 100 $\mu$m or less.

The present invention relates to a method for providing a polytetrafluoroethylene tube, in which, in a case where a surface shape of an inner surface of the tube is measured, when a direction of the measurement is parallel to a longitudinal direction of the tube and the measurement is performed in a forward direction and a reverse direction, a variation rate between a size of a periodic step measured in the forward direction and a size of the periodic step measured in the reverse direction is 5% or more, and the tube is provided in a state of being in contact with a mandrel.

## EFFECT OF INVENTION

According to the above configuration, it is possible to provide a tube having an appropriate tensile strength and excellent flexibility in the entire tube and being excellent in insertion property. The tube according to the present invention has an appropriate strength even if the tube is thin as a liner tube, and is suitable as a tubular liner tube or the like that requires flexibility and a good insertion feeling.

## BRIEF DESCRIPTION OF DRAWINGS

Fig. 1 is a diagram illustrating a form of a tube according to the present invention.
Fig. 2 illustrates schematic views each illustrating an example of a cross section of the tube according to the present invention.
Fig. 3 is a schematic view illustrating an example of a cross section of the tube according to the present invention.
Fig. 4 is a diagram illustrating an example of a film used for the tube according to the present invention.
Fig. 5 is a diagram illustrating parameters related to the tube according to the present invention.
Fig. 6 is a diagram illustrating an example of a film winding structure of the tube according to the present invention.
Fig. 7 is a diagram illustrating a roughness curve and parameters.

## EMBODIMENTS FOR CARRYING OUT THE INVENTION

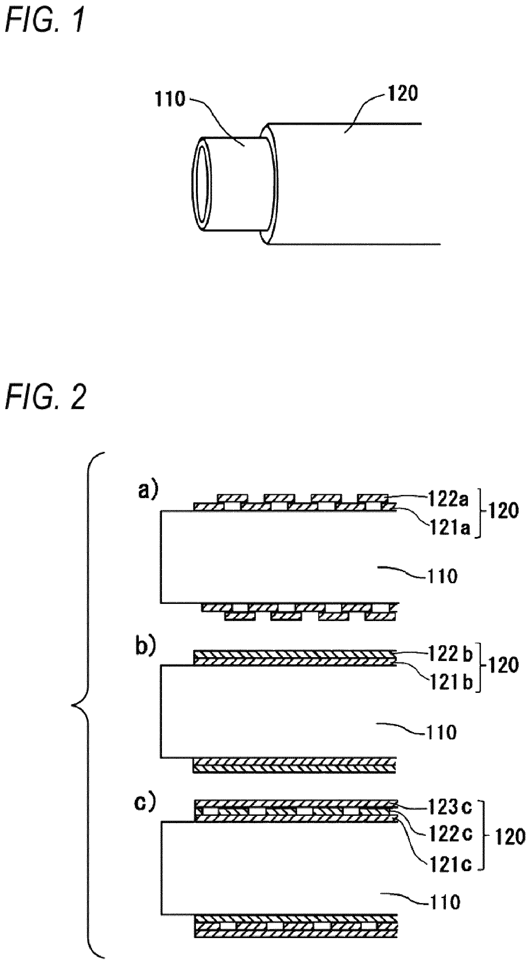

Fig. 1 is a diagram illustrating a tube according to the present invention in a state of being covered on a mandrel. The tube according to the present invention can be provided in a state in which a tube 120 is covered on an outer periphery of a mandrel 110. An outer surface of the tube 120 may be etched. The tube 120 has a cylindrical form made of a resin containing PTFE, and the tube 120 is preferably a tube in which at least one film is spirally wound, and which is formed by the at least one film in a cylindrical shape without a gap. Here, "formed without a gap" means, for example, a state in which an outer peripheral surface of the mandrel 110 is completely covered with one or more films when the tube is disposed outside the mandrel 110.

Fig. 2 illustrates schematic views each illustrating a part of a cross section along a longitudinal direction of a tube formed by spirally winding a film, which is an example of the tube according to the present invention. Fig. 2 illustrates an example of the tube 120 configured by at least two layers. a) of Fig. 2 illustrates a case in which the tube 120 is configured by two layers, and a film 121a serving as a first layer is spirally wound around the outer periphery of the mandrel 110 at intervals. A film 122a serving as a second layer is spirally wound outside the film 121a in a manner of filling the interval formed by the film 121a serving as the first layer. Accordingly, the outer peripheral surface of the mandrel 110 is completely covered with the two layers of films. b) of Fig. 2 illustrates another example of the tube 120 including two layers. A film 121b serving as a first layer is spirally wound on the mandrel 110 without a gap, and a film 122b serving as a second layer is spirally wound outside the film 121b without a gap. c) of Fig. 2 illustrates an example of a case in which the tube 120 includes three layers. A film 121c serving as a first layer is spirally wound on the outer periphery of the mandrel 110 without a gap, a film 122c serving as a second layer is spirally wound outside the film 121c at intervals, and a film 123c serving as a third layer is spirally wound outside the film 122c without a gap. The tube according to the present invention may include, for example, four or more layers.

In the tube according to the present invention, a periodic step according to windings of a film is formed on an inner surface of the tube before sintering. The periodic step is a step that includes a common pattern shape at intervals according to a certain period, and the period at which the step appears may be continuously modulated according to a certain rule. The tube according to the present invention is preferably obtained by spirally winding a film on a mandrel, and then heating and sintering the film at a temperature equal to or higher than a melting point of PTFE. By performing sintering, the wound film constituting the tube is melted, and the inner surface of the tube comes into close contact with the mandrel. A close contact strength varies depending on conditions for sintering the tube, and the like, and is preferably a strength that does not cause the periodic step to be lost due to transferring of a mandrel surface.

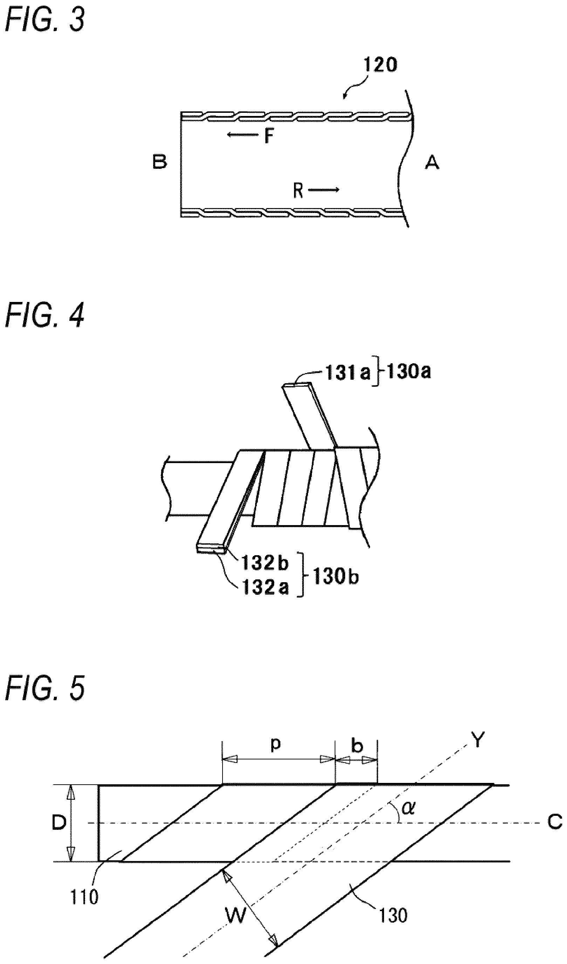

In the tube according to the present invention, in a case where a surface shape of the inner surface of the tube is measured by using a contact type surface roughness meter, a detected size of the periodic step varies depending on a direction of the measurement. Fig. 3 is a diagram schematically illustrating an example of a film winding structure of the tube according to the present invention. Assuming that a direction from an end on an A side to an end on a B side of the tube 120 is a forward direction F, and a direction from the end on the B side to the end on the A side of the tube, which is opposite to the forward direction F, is a reverse direction R, when the surface shape of the inner surface of the tube is measured in a direction in parallel to the longitudinal direction of the tube by using a contact type surface roughness meter, in the tube according to the present invention, a size of the periodic step measured in the forward direction F is different from a size of the periodic step measured in the reverse direction R, and a variation rate is preferably 5% or more, more preferably 10% or more, and still more preferably 20% or more. The difference in the size of the periodic step is considered to be caused by a difference in a portion where the periodic step formed on the inner surface of the tube and a tip of a stylus of the roughness meter are in contact with each other between the measurement in the forward direction F and the measurement in the reverse direction R in the surface shape measurement of the inner surface of the tube. Here, the variation rate between the size of the periodic step measured in the forward direction and the size of the periodic step measured in the reverse direction refers to a value obtained by dividing an absolute value of a difference value between the sizes of the step by an average value of the sizes of the step. In the tube according to the present invention, a width of the periodic step is preferably 100 $\mu$m or less.

The tube according to the present invention is preferably high in initial tensile strength when a force for stretching the tube in the longitudinal direction is applied to the tube. Due to the high tensile strength, even when a treatment tool or the like is strongly rubbed against the inside of the tube when the treatment tool or the like is inserted into the inside of the tube, the tube does not stretch and become wrinkled, and can maintain high slidability and insertion feeling. Regarding the tensile strength of the tube according to the present invention, in a tensile test according to JIS K7127-1999, a 20% strain tensile strength of the tube is preferably 100 N/mm$^2$ or more.

An average wall thickness of the tube serving as a PTFE liner tube is generally required to be thin. A thickness of the tube according to the present invention is preferably 5 $\mu$m or more and 75 $\mu$m or less, more preferably 5 $\mu$m or more and 50 $\mu$m or less, and still more preferably 5 $\mu$m or more and 40 $\mu$m or less.

The tube according to the present invention has an inner diameter of 0.05 mm or more and 10 mm or less. The inner diameter depends on a use for which the tube is used, but in order for an effect of the tube according to the present invention to be particularly exhibited, the inner diameter is preferably 0.075 mm or more and 8 mm or less, and more preferably 0.1 mm or more and 5 mm or less.

A method for producing the tube according to the present invention will be described with reference to an example in which the film is spirally wound around the mandrel.

As the mandrel used for producing the tube according to the present invention, it is possible to use a pipe made of a metal such as SUS, copper, silver, brass, aluminum, or nickel titanium, a pipe made of a resin such as PTFE or polyether ether ketone (PEEK), a metal wire made of SUS, copper, silver, aluminum, nickel titanium or the like, a wire material obtained by plating the metal wire with silver or nickel, or a resin wire made of PTFE or PEEK. A cross-sectional shape of the mandrel is usually a circular shape (including a substantially circular shape), but may be a quadrangular shape, a polygonal shape, or the like, or may be partially changed in shape, as necessary. Since the tube is in contact with the outer peripheral surface of the mandrel, the surface of the mandrel preferably has a shape necessary for the shape of the inner surface of the tube.

When a mandrel having a circular cross-sectional shape is used, an outer diameter of the mandrel can be appropriately selected according to the inner diameter of the tube, and for example, a mandrel having an outer diameter of 0.05 mm or more and 10 mm or less can be used. It is preferable to use a mandrel having a tensile elongation at break of 5% or more such that the mandrel can be stretched to reduce an outer diameter and can be removed from the tube at a stage in which the mandrel becomes unnecessary in a producing process. In order to prevent breakage during stretching, a tensile strength at break is preferably 75 MPa to 600 MPa.

Fig. 4 is a diagram illustrating an example of the film used for the tube according to the present invention, and illustrates an example in which a second layer 130a of the tube is configured by one film 131a made of PTFE, and a first layer 130b is formed by laminating one film 132a made of PTFE and one film 132b made of a thermoplastic fluororesin. The film used for the tube according to the present invention is not limited to a laminate of two resin films such as the 130b, and a laminate of three or more resin films can also be used. A plurality of laminated resin films may or may not be integrated. In the present invention, "one film" includes a case in which two or more resin films are laminated and wound around a mandrel (in some cases, on a layer of a tube) at the same angle, as in the second layer 130b illustrated in Fig. 4.

The film comprising PTFE used for the tube according to the present invention is preferably comprising high density PTFE. The use of high density PTFE is advantageous for achieving airtightness and strength of a product using the tube as an inner layer. The film comprising high density PTFE can be produced, for example, as follows. A preformed article, which is obtained by mixing a PTFE resin fine powder and an auxiliary agent (a lubricant such as solvent naphtha or white oil) and compressing the mixture, is put into an extruder to be molded into a film shape, and then a molded body is dried. During drying, the auxiliary agent contained in the filmlike molded body is volatilized, and an unsintered PTFE film having pores in the film is obtained. When the unsintered PTFE film is heated to a temperature equal to or higher than a melting point and sintered, the pores in the film disappear, and a high density PTFE film is obtained. At this time, the film can be further compressed through a pressure roll. The high density PTFE film can also be obtained by pressurizing a PTFE film having a porous structure produced by uniaxially or biaxially expanding the unsintered PTFE film while heating at a temperature equal to or lower than the melting point. The pressurized PTFE film may be used after being sintered. The produced film is generally used after being slit to an appropriate width. The PTFE film having a porous structure described above may be caused to have a high density after being spirally wound around an outer periphery of a mandrel, for example. In this case, after the PTFE film having a porous structure is wound around the outer periphery of the mandrel, the mandrel is caused to pass through a ring-shaped die to compress the PTFE having a porous structure, and then the PTFE can be converted into high density PTFE.

The film used for the tube according to the present invention may contain a filler or other resins as necessary. Examples of the filler include a filler made of carbon, a metal oxide such as alumina, and a resin, and examples of the other resins include a thermoplastic fluororesin. The filler or the other resins may be used alone or in combination.

The PTFE film used for the tube according to the present invention may be implemented by laminating the film made of PTFE and the film made of the thermoplastic resin as described above. A fluororesin used as a material for the film made of a thermoplastic fluororesin is preferably a resin having a melting point lower than a crystal melting point of PTFE, such as tetrafluoroethylene-hexafluoropropylene copolymer (FEP) and tetrafluoroethylene-perfluoroalkyl vinyl ether copolymer (PFA). When the PTFE film is formed by laminating the film made of PTFE and the film made of a thermoplastic fluororesin, a ratio between (sums of) thicknesses of the respective films made of the resins in one PTFE film is preferably in a range of (thickness of resin film made of PTFE/thickness of resin film made of thermoplastic fluororesin) = 10/1 to 1/1. In the tube according to the present invention, it is preferable that PTFE is a main component, and here, the main component means that a volume ratio exceeds half of the entire tube.

A thickness of the film used for the tube according to the present invention is one of factors that influence the size of the periodic step on the inner surface of the tube, and can be determined according to required characteristics of the tube. For example, the thickness of the film may be 2 $\mu$m or more and 25 $\mu$m or less. If the thickness of the film is small, the step on the inner surface of the tube can be easily adjusted, but if the thickness is too small, handling becomes difficult, and the film may be broken or wrinkled during work. If the thickness of the film is large, the step on the inner surface of the tube becomes large, and at the same time, it becomes difficult to make the wall thickness of the tube thin.

The width of the film used for the tube according to the present invention can be appropriately determined according to the inner diameter of the tube, a necessary thickness of the tube, a winding angle of the film, and the like. Fig. 5 is a diagram illustrating parameters related to the film winding structure of the tube according to the present invention. When the outer diameter of the mandrel 110 (the inner diameter of the tube) is defined as D, a winding angle of a film is defined as $\alpha$, a traveling distance, that is, a pitch when a film 130 is wound for one winding is defined as P, and an overlapping amount of the wound film 130 is defined as b, a width W of the film 130 can be obtained by the following formula. Here, the winding angle $\alpha$ of the film is an angle sandwiched between a center axis C of the tube and a center line Y of the width of the film, and is an angle larger than 0 degrees and smaller than 90 degrees.

$$p = \pi D \cos\alpha$$

$$W = P\sin\alpha + b$$

In the present invention, the number of windings (the number of laps) of the film is obtained by the following formula. The width of the film 130 is defined as W, and the overlapping amount of the film 130 is defined as b.

$$\text{Number of windings (number of laps) of film} = 1 + (b/W)$$

When the film 130 is wound at intervals, the overlapping amount of the film 130 is represented by a minus amount, and the number of windings of the film is a number less than 1.

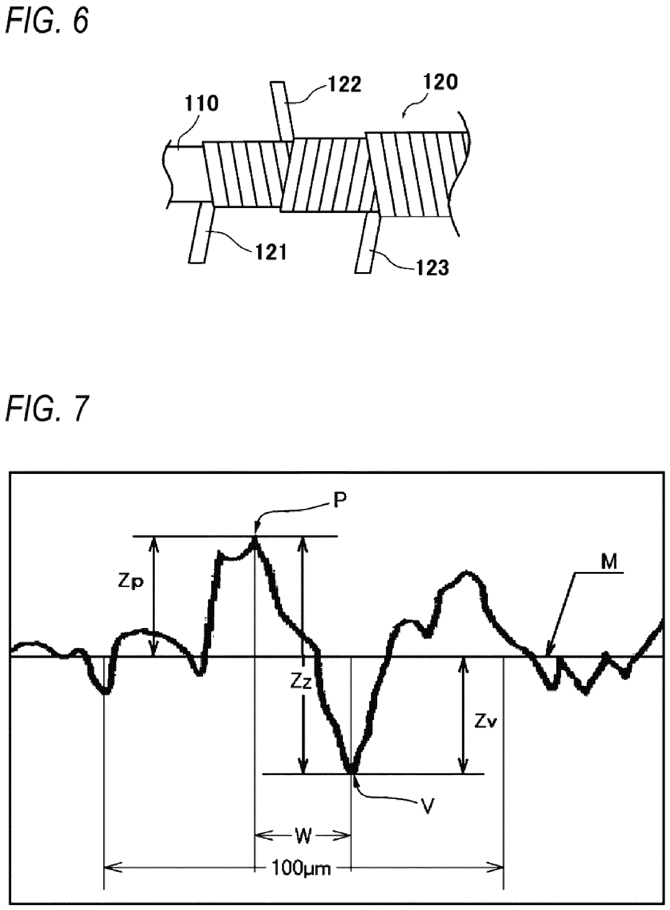

Fig. 6 is a diagram illustrating an example of a film winding structure of the tube according to the present invention. In the example illustrated in Fig. 6, the tube includes three layers each of which is configured by one film. The film of each layer is wound clockwise (Z-winding) or counterclockwise (S-winding). In the example illustrated in Fig. 6, a first layer 121 of the tube 120 is wound clockwise. A second layer 122 is wound in a film winding direction different from that of the first layer 121, that is, counterclockwise. A third layer 123 is wound in the same film winding direction as that of the first layer, that is, clockwise. In an elongated body according to the present invention, it is preferable that one or more films are spirally wound clockwise and one or more films are spirally wound counterclockwise opposite to the clockwise wound film.

Each layer of the tube according to the present invention is preferably wound at a film winding angle of 3 degrees to 30 degrees, more preferably 3 degrees to 20 degrees. The winding angles of all the layers constituting the tube do not need to be the same, and the layers may be wound at different winding angles. The films of the respective layers of the tube are preferably wound in a manner that ends of the films overlap each other, but can also be formed in a manner of completely covering the outer periphery of the mandrel by laminating layers that are wound with a gap. For example, Fig. 3 described above illustrates an example in which the film serving as the first layer of the tube is wound with the number of windings of 1.5 laps (that is, the number of windings with which half of the width of the film overlaps an adjacent film). The overlapping of the ends of the film is one of factors that influence the size of the periodic step on the inner surface of the tube.

The tube according to the present invention has been described above by taking a lapping tube as an example, but the tube according to the present invention can also be obtained in a case in which a pattern serving as a step is provided on an inner surface of an extrusion tube.

The present invention also includes one obtained by using the tube according to the present invention as a base material and further processing on the tube according to the present invention. At this time, the tensile test of the tube according to the present invention is preferably performed in a state of a PTFE tube alone, which is not processed.

The method for producing a tube according to the present invention will be described in more detail in the following examples. The following examples are merely examples of the present invention, and are not intended to limit the content of the present invention.

**EXAMPLES**

<Measurement of Shape of Inner Surface of Tube>

The measurement of the shape of the inner surface of the tube was performed at a portion around a center in the longitudinal direction of the tube (a product when the product is produced by using the tube).

In order to make an insertion feeling close to an insertion feeling of the product produced by using this tube, a tube that was cut open was disposed on a soft sheet. As the soft sheet, for example, an adhesive tape on which an adhesive is laminated can be used. Here, the measurement was performed by fixing the tube by using PKH-20 manufactured by 3M Company. A contact type surface roughness measuring instrument was used for the shape measurement. The measurement was performed according to JIS B0601-2001 by using a surftest SJ-410 manufactured by Mitutoyo Corporation. Measurement conditions were as follows.

Stylus used: tip radius 5 $\mu$m, tip angle 90°
Measurement speed: 0.5 mm/sec
Measuring force: 4 mN
Measured data was represented as a roughness curve under the following conditions.
Filter used: GAUSS
Cutoff value $\lambda$c: 0.25 mm
Cutoff value $\lambda$s: 2.5 $\mu$m

Fig. 7 illustrates an example of the roughness curve obtained by the shape measurement (a part, in the vicinity of a peak, of the periodic step). An average line M for obtaining the roughness curve is defined as an X axis, and in a part of a curve sandwiched between two adjacent intersections when the roughness curve is cut along the X axis, a side upper than the average line M is defined as a peak and a side lower than the average line M is defined as a valley. A center of an interval on the X axis between vertices of peaks (a peak P and a valley V) of a periodic step confirmed by the roughness curve is defined as a center of an evaluation length, and in the roughness curve within the range of the evaluation length of 100 $\mu$m, a sum of a height $Zp$ of a highest peak and a depth $Zv$ of a deepest valley is defined as a size $Zz$ of the periodic step.

The size $Zz$ of the periodic step is preferably an average value of three consecutive points, and more preferably an average value of five consecutive points. A variation rate between a size $Zz1$ of the periodic step measured in the forward direction and a size $Zz2$ of the periodic step measured in the reverse direction is calculated by dividing a difference value between the sizes of the steps in both directions by an average value of the sizes of the steps in both directions.

$$\text{Variation rate\%} = (|Zz1 - Zz2|)/((Zz1 + Zz2)/2) \times 100 \qquad \text{Formula (1)}$$

The interval on the X-axis between the vertices of the peaks (the peak P and the valley V) of the periodic step confirmed by the roughness curve is defined as the width W of the periodic step. The width of the periodic step is preferably an average value of three consecutive points, and more preferably an average value of five consecutive points.

<Measurement of Dimension of Tube>

The inner diameter, the outer diameter, and the wall thickness of the tube are measured by using a microscope in a cross section perpendicular to the longitudinal direction of the tube, or the inner diameter is used by using a pin gauge, and the outer diameter is measured by using a dial gauge with the pin gauge inserted. When a microscope is used, the measurement is performed in four or more directions in a manner that the measurement can be performed as evenly as possible from each direction in the same cross section, and an arithmetic average value is obtained. When a pin gauge and a dial gauge are used, the measurement is performed in four directions as evenly as possible, and an arithmetic average value is obtained. It is preferable to perform the measurement on cross sections of plural parts of the same sample to obtain an arithmetic average value. When the inner diameter of the tube is large, the tube may be cut open to form a film, and the dimensions may be measured with a vernier caliper, a dial gauge, or the like.

The dimensions of the tube are preferably measured at a portion around the center in the longitudinal direction of the tube (a product when the product is produced by using the tube). For example, regarding the position of the tube, it is conceivable to define a portion around the center in the longitudinal direction of the product as a center of a range for measuring dimensions of the tube, measure dimensions at a plurality of positions in a range of about 25 cm before and after the center, and obtain an arithmetic average value thereof.

<Tensile Test>

A tensile test of the tube was performed according to JIS K7127-1999. A test piece was a tube cut to a length of about 8 cm to 10 cm or more. The test piece is preferably implemented in a manner that a portion of the tube that is around the center in the longitudinal direction of the tube (a product when the product is produced by using the tube) can be disposed at a center of a chuck. The test piece was set on a chuck of a tensile tester, and the tensile test was performed with a distance between chucks as a distance between sample gauge lines. As the tensile tester, Autograph AGS-1kNX model manufactured by Shimadzu Corporation was used, and the test was performed in an environment of 23°C $\pm$ 2°C under conditions of an initial distance between chucks of 50 mm and a test speed of 200 mm/min. In the tensile test, a tensile stress when a strain was 20% was defined as a 20% strain tensile strength. The 20% strain tensile strength is preferably 100 N/mm$^2$ or more.

Example 1

A stainless steel wire having an outer diameter of 0.45 mm was prepared as a mandrel, and a film made of PTFE having a thickness of 7 $\mu$m was prepared as a film. On an outer periphery of the mandrel, as a first layer, the PTFE film was spirally wound clockwise with the number of windings of 1.5 laps, and as a second layer, the PTFE film was spirally wound counterclockwise with the number of windings of 1.3 laps, and the two layers were laminated. The mandrel on which the films were laminated was caused to pass through an oven heated to 370°C, and then air-cooled, and wound around a reel. The mandrel was cut to an appropriate length, only the mandrel was stretched to reduce the outer diameter, and then the mandrel was taken out to obtain a tube. Properties of the obtained tube were summarized in Table 1.

Example 2

A stainless steel pipe having an outer diameter of 1. 51 mm was prepared as a mandrel. A film (a laminated film) obtained by laminating a film made of PTFE having a thickness of 6 $\mu$m and a film made of PFA having a thickness of 2 $\mu$m was prepared as a film. On an outer periphery of the mandrel, as a first layer, the laminated film was spirally wound counterclockwise with the number of windings of 1.5 laps in a manner that a PTFE side of the laminated film faces the mandrel, and as a second layer, the laminated film was spirally wound clockwise with the number of windings of 1.5 laps on the first layer, and then the two layers were laminated. The mandrel on which the films were laminated was caused to pass through an oven heated to 370°C, and then air-cooled, and wound around a reel. The mandrel was cut to an appropriate length, only the mandrel was stretched to reduce the outer diameter, and then the mandrel was taken out to obtain a tube. Properties of the obtained tube were summarized in Table 1.

Comparative Example 1

To 100 parts by weight of a PTFE fine powder, 15 parts by weight of an auxiliary agent was added, and the mixture was put into a preforming machine to prepare a preformed body. The preformed body was put into an extruder and extruded into a cylindrical shape with a ram speed of 3 mm/min. After drying, the molded PTFE was heated and sintered in an oven at 430°C, and then air-cooled to obtain a PTFE tube having an inner diameter of 1.5 mm and a wall thickness of 0.025 mm.

For each of the tubes according to the examples and the comparative example, a shape of an inner surface of the tube was measured, the size Zz1 of a periodic step measured in a forward direction and the size Zz2 of the periodic step measured in a reverse direction were obtained, and a variation rate was calculated. Each of the tube was used as a test piece, and a tensile test according to JIS K7127-1999 was performed to confirm a 20% strain tensile strength. Results are illustrated in Table 1.

[Table 1]

|  | Tube inner diameter (mm) | Tube wall thickness (mm) | Variation rate of size of step (%) | Width of step ($\mu$m) | 20% strain tensile strength (N/mm$^2$) |
|---|---|---|---|---|---|
| Example 1 | 0.45 | 0.015 | 38.1 | 51.3 | 120.7 |
| Example 2 | 1.51 | 0.012 | 13.8 | 24.2 | 269.4 |
| Comparative Example 1 | 1.50 | 0.025 | - | - | 94.0 |

Each of the tubes according to the examples has a variation rate of the size of the periodic step on the inner surface of the tube of 5% or more. The periodic step did not appear on the inner surface of the tube according to the comparative example. The tubes according to the examples were tubes with strength and flexibility, and were excellent in insertion property when a treatment tool or the like was inserted into the tube, and were excellent in insertion feeling such as straightness and operability of the treatment tool or the like.

**INDUSTRIAL APPLICABILITY**

The tube according to the present invention has an appropriate tensile strength and flexibility in the entire tube while being thin, and is excellent in internal insertion property. The tube according to the present invention has a sufficient strength even if the tube is thin, and is suitable as a tubular liner tube or the like requiring flexibility and a good insertion feeling.

**REFERENCE SIGNS LIST**

110: mandrel
120: tube
121: first layer
122: second layer
123: third layer

130: film

**Claims**

1. A polytetrafluoroethylene tube, the tube comprising polytetrafluoroethylene, wherein

   in a case where a surface shape of an inner surface of the tube is measured,
   when a direction of the measurement is parallel to a longitudinal direction of the tube and the measurement is performed in a forward direction and a reverse direction,
   a variation rate between a size of a periodic step measured in the forward direction and a size of the periodic step measured in the reverse direction is 5% or more.

2. The polytetrafluoroethylene tube according to claim 1, wherein
   the tube is a tube in which at least one film is spirally wound, and which is formed by the at least one film without a gap.

3. The polytetrafluoroethylene tube according to any one of claims 1 or 2, wherein
   in the tube, one or more films are spirally wound clockwise, and one or more films are spirally wound counterclockwise.

4. The polytetrafluoroethylene tube according to any of claims 1 to 3, wherein
   a 20% strain tensile strength of the tube obtained by a tensile test according to JIS K7127-1999 is 100 N/mm$^2$ or more.

5. The polytetrafluoroethylene tube according to any one of claims 1 to 4, wherein
   the tube has an average thickness of 5 μm or more and 75 μm or less.

6. The polytetrafluoroethylene tube according to any one of claims 1 to 5, wherein
   the tube has an inner diameter of 0.05 mm or more and 10 mm or less.

7. The polytetrafluoroethylene tube according to any one of claims 1 to 6, wherein
   a width of the periodic step is 100 μm or less.

8. A method for providing a polytetrafluoroethylene tube, wherein

   in a case where a surface shape of an inner surface of the tube is measured,
   when a direction of the measurement is parallel to a longitudinal direction of the tube and the measurement is performed in a forward direction and a reverse direction,
   a variation rate between a size of a periodic step measured in the forward direction and a size of the periodic step measured in the reverse direction is 5% or more, and
   the tube is provided in a state of being in contact with a mandrel.

9. The polytetrafluoroethylene tube according to any one of claims 1 to 7, wherein
   an outer surface of the tube is etched.

*FIG. 1*

*FIG. 2*

*FIG. 3*

*FIG. 4*

*FIG. 5*

*FIG. 6*

*FIG. 7*

## INTERNATIONAL SEARCH REPORT

| | International application No. |
|---|---|
| | **PCT/JP2021/036663** |

**A. CLASSIFICATION OF SUBJECT MATTER**

*B29D 23/20*(2006.01)i; *B29K 27/18*(2006.01)n; *B29L 23/00*(2006.01)n; *A61M 25/00*(2006.01)i; *B29C 53/60*(2006.01)i; *B29C 70/04*(2006.01)n; *B29C 70/32*(2006.01)i; *F16L 11/16*(2006.01)i; *F16L 11/24*(2006.01)i
FI: F16L11/24; A61M25/00 500; B29C53/60; B29C70/32; B29D23/20; F16L11/16; B29C70/04; B29K27:18; B29L23:00

According to International Patent Classification (IPC) or to both national classification and IPC

**B. FIELDS SEARCHED**

Minimum documentation searched (classification system followed by classification symbols)
F16L11/24; A61M25/00; B29C53/60; B29C70/32; B29D23/20; F16L11/16; B29C70/04; B29K27/18; B29L23/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2010-500106 A (GORE ENTERPRISE HODLINGS, INC.) 07 January 2010 (2010-01-07) paragraphs [0026]-[0033], fig. 1-2 | 1-2, 5-8 |
| Y | | 3-4, 9 |
| Y | JP 11-512329 A (W. L. GORE & ASSOCIATES, INC.) 26 October 1999 (1999-10-26) page 12, lines 2-8, fig. 1-2 | 3-4, 9 |
| Y | JP 2005-024931 A (JAPAN GORE TEX INC.) 27 January 2005 (2005-01-27) claims, paragraph [0105] | 4, 9 |
| Y | JP 2002-085334 A (FUJI PHOTO OPTICAL CO., LTD.) 26 March 2002 (2002-03-26) paragraphs [0021]-[0044], fig. 1-11 | 9 |
| A | JP 2006-323017 A (JAPAN GORE TEX INC.) 30 November 2006 (2006-11-30) entire text, all drawings | 1-9 |
| A | JP 2004-106349 A (JAPAN GORE TEX INC.) 08 April 2004 (2004-04-08) entire text, all drawings | 1-9 |

☑ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **09 December 2021** | **21 December 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

| | International application No. |
|---|---|
| | **PCT/JP2021/036663** |

**C.    DOCUMENTS CONSIDERED TO BE RELEVANT**

| Category* | Citation of document, with indication, where appropriate, of the relevant passages | Relevant to claim No. |
|---|---|---|
| A | JP 2015-524348 A (W. L. GORE & ASSOCIATES, INC.) 24 August 2015 (2015-08-24) entire text, all drawings | 1-9 |

Form PCT/ISA/210 (second sheet) (January 2015)

**INTERNATIONAL SEARCH REPORT**
Information on patent family members

International application No.

**PCT/JP2021/036663**

| Patent document cited in search report | | | Publication date (day/month/year) | Patent family member(s) | | | Publication date (day/month/year) |
|---|---|---|---|---|---|---|---|
| JP | 2010-500106 | A | 07 January 2010 | US | 2010/0049123 | A1 | |
| | | | | paragraphs [0033]-[0040], fig. 1-2 | | | |
| | | | | WO | 2008/021003 | A1 | |
| | | | | EP | 2412399 | A1 | |
| JP | 11-512329 | A | 26 October 1999 | WO | 1997/010871 | A1 | |
| | | | | page 7, line 27 to page 8, line 4, fig. 1-2 | | | |
| | | | | EP | 851777 | A1 | |
| JP | 2005-024931 | A | 27 January 2005 | US | 2006/0154010 | A1 | |
| | | | | claims, paragraph [0110] | | | |
| | | | | WO | 2005/003866 | A1 | |
| | | | | EP | 1640818 | A1 | |
| JP | 2002-085334 | A | 26 March 2002 | US | 2002/0032370 | A1 | |
| | | | | paragraphs [0041]-[0066], fig. 1-11 | | | |
| | | | | EP | 1188976 | A2 | |
| JP | 2006-323017 | A | 30 November 2006 | US | 2009/0097890 | A1 | |
| | | | | entire text, all drawings | | | |
| | | | | WO | 2006/123620 | A1 | |
| | | | | EP | 1887439 | A1 | |
| JP | 2004-106349 | A | 08 April 2004 | (Family: none) | | | |
| JP | 2015-524348 | A | 24 August 2015 | US | 2014/0142682 | A1 | |
| | | | | entire text, all drawings | | | |
| | | | | WO | 2014/026174 | A1 | |
| | | | | EP | 2882464 | A1 | |
| | | | | CN | 104519922 | A | |
| | | | | KR | 10-2015-0042187 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000316977 A
- JP 2013176583 A
- JP 2004340364 A
- JP 6244490 B